Europäisches Patentamt

European Patent Office

Office européen des brevets

(19)

(11) Publication number: 0 101 200
A2

# EUROPEAN PATENT APPLICATION

(12)

(21) Application number: 83304186.6

(22) Date of filing: 19.07.83

(51) Int. Cl.³: C 07 G 17/00
A 61 K 35/12

(30) Priority: 20.07.82 GB 8220915

(43) Date of publication of application:
22.02.84 Bulletin 84/8

(84) Designated Contracting States:
AT BE CH DE FR GB IT LI LU NL SE

(71) Applicant: BIO-COM Inc.
Apartado 850 Calle Aquilino de la Guardia No. 8
Panama 1(PA)

(72) Inventor: Viza, Dimitri
62 Rue Brancion
Paris 75015(FR)

(72) Inventor: Adamopoulos, Dimitri
5 Kyprou Neon Psychikon
Athens(GR)

(72) Inventor: Pizza, Gian Carlo
Via Bezzecca No. 5
Bologna 40141(IT)

(74) Representative: Woods, Geoffrey Corlett et al,
J.A. KEMP & CO. 14 South Square Gray's Inn
London WC1R 5EU(GB)

(54) Transfer factor for use in treating viral infections.

(57) Transfer factor specific to a given virus is prepared by immunising a mammal with respect to the virus and obtaining the transfer factor thus-produced specific to the virus from the immunised mammal, for example in the form of a dialysate of cells of lymphoid origin or of liver cells taken from the immunised mammal. The transfer factor can then be used to combat a viral infection by administering to a patient transfer factor specific to the virus to which the infection is attributable.

EP 0 101 200 A2

## DESCRIPTION

TITLE: TRANSFER FACTOR FOR USE IN TREATING VIRAL INFECTIONS

This invention relates to virus-specific transfer factor and to its use in therapy.

Cutaneo-mucosal infections due to both types of Herpes Simplex Virus (HSV) are very often resistant to the usual treatment of viral infections. HSV 1 is, in general, responsible for lesions of the face and keratitis of the eye, and HSV 2 is usually responsible for genital lesions apparently sexually transmitted. However, this distinction is not absolute.

It has now been found that HSV 1 and HSV 2 infections can be combated or prevented by use of transfer factor specific to HSV 1 or HSV 2. This transfer factor is obtained by immunising a mammal with a HSV 1 or HSV 2 antigen. It has also been found that viral-specific transfer factor produced by immunising a mammal with antigens of other viruses can be employed in the treatment of other viral infections. The transfer factor produced has a clear specificity and is capable of transferring its information _in vivo_ to other mammals or _in vitro_ to non-sensitised human or animal lymphocytes. Thus, the transfer factor specific to a given virus is capable of transferring cell-mediated immunity in respect of the virus to naive (non-sensitised) lymphocytes _in vitro_ and to non-sensitised mammals _in vivo_.

Accordingly, the present invention provides transfer factor specific to a given virus, the transfer factor having been obtained by immunising a mammal with respect to the virus and obtaining the transfer factor thus-produced specific to the virus from the immunised mammal.

Preferably, the virus is a herpesvirus, myxovirus, hepatitis virus, arbovirus or pathogenic animal virus. The herpesvirus is preferably herpes simplex virus 1, herpes simplex virus 2, cytomegalovirus, herpes zoster virus (for example causing chickenpox). The myxovirus may be a paramyxovirus or orthomyxovirus, and is preferably a measles virus, an influenza virus or a rubella virus. The hepatitis virus is preferably hepatitis B virus. The arbovirus may be an arbovirus B such as yellow fever or dengue fever virus. The pathogenic animal virus may be the virus causing african swine fever, foot and mouth disease, Newcastle disease or vesicular stomatitis. The virus may also be the virus causing molluscum contagiosum or warts.

Any suitable mammal, for example guinea pigs or calves, can be immunised with respect to the virus. Preferably the animal is healthy and does not show a positive reaction for the virus when tested prior to immunisation. Immunisation is generally achieved by use of an antigenically active form of the virus or an antigenic determinant of the virus. The wild-type virus itself can be used but it is preferable to use an attenuated or inactivated strain. Where the antigenic determinant of a virus is known, it may need to be coupled to a carrier molecule to exhibit immunogenicity. In relation to HSV, an HSV antigen may be used.

Whatever is used to immunise the mammal, it is generally injected into the mammal. Typically several injections over a period of time are required. The point at which the mammal has acquired the necessary degree of immunisation can be evaluated by, for example, a skin test, leucocyte migration inhibition test and/or lymphocyte transformation test.

The transfer factor specific to the virus can be obtained from the immunised mammal in a variety of ways. Milk and urine contain the transfer factor. It is also possible to draw blood from the immunised mammal and obtain the transfer factor from its serum or plasma or by using plasmapheresis. Alternatively, cells of lymphoid origin can be extracted from the immunised mammal and the transfer factor obtained from the extracted cells. The cells of lymphoid origin may be peripheral blood lymphocytes or cells from lymphatic tissues such as the spleen or the lymph nodes. The peripheral blood lymphocytes may be obtained by leucopheresis or by repeated bleedings, i.e. bleeding an immunised mammal every two to three months, for example. It may be necessary to kill the immunised mammal to obtain the transfer factor.

The transfer factor is preferably obtained from cells of lymphoid origin or liver cells extracted from the immunised mammal by disrupting the cells and dialysing their contents. The cells may be disrupted by repeated freeze-thawing techniques or by means of a homogeniser or sonication. The disrupted cells can then be dialysed through a dialysis bag (Visking cellulose) against distilled water or a vacuum. Alternatively, the disrupted cells can be introduced into an ultra-filtration apparatus possessing, for example, an Amicon filter. The dialysate or filtrate thus obtained comprises the transfer factor specific to the virus.

Generally, conditions are chosen such that a cell fraction comprising the transfer factor is obtained which contains substantially no molecules with a molecular weight of more than 12,000. This ensures that the cell fraction does not contain any viral genomes.

The cell fractions contain, in addition to the transfer factor (inducer factor), a suppressor factor capable of inducing immunosuppression or immunoregulation of the immune response to an antigen in a subject. The suppressor factor can therefore induce a state of immunological tolerance to an antigen. Cell fractions which contain the transfer factor but do not contain suppressor factor can be obtained by one of two basic techniques:

1) after obtaining the cells of lymphoid origin or liver cells, either removing the T-suppressor cells or collecting the T-helper cells, and then obtaining the cell fraction, for example by dialysis, as described above; or

2) obtaining the desired cell fraction as above from all the cells of lymphoid origin or liver cells and either removing the suppressor factor from the cell fraction or collecting the transfer factor, and obtaining a fraction containing the transfer factor but not the suppressor factor, for example by immunoadsorption of the transfer factor and subsequent elution of the transfer factor.

In method 1) the T-suppressor cells can be removed or the T-helper cells collected by immunoadsorption. Similarly in method 2), since the suppressor factor binds to the antibody to the antigen to which the suppressor factor is specific and since the transfer factor binds to the antigen to which the transfer factor is specific, immunoadsorption techniques can also be used to remove the suppressor factor or collect the transfer factor.

The transfer factor can be replicated by a lymphoblastoid cell line as described in G.B. Patent Specification No. 1,443,948. Briefly, a lymphocyte dialysate comprising the transfer factor is filtered through a Milipore membrane (0.22 μm). The filtrate is added to a culture of lymphoblastoid cells at a concentration of $5 \times 10^5$ cells/ml and at a ratio of 1 unit of dialysate (from $10^8$ cells) per $10^8$ cells. The cells thus induced are maintained in culture until a number sufficient for extraction of further transfer factor specific to HSV 1 or HSV 2 are obtained, usually

more than $10^8$ cells.

The preferred lymphoblastoid cell line for use in this replication procedure is the known LDV/7 cell line. This cell line is described in G.B. Patent Specification No. 1,592,954.

The transfer factor prepared by the process of the invention can be lyophilised, for example, for storage. When required for use it can then be resuspended in a suitable volume of a physiologically acceptable diluent so as to form an isotonic solution.

The invention therefore also provides a pharmaceutical composition comprising, as active ingredient, transfer factor according to the invention specific to a virus, together with a physiologically acceptable carrier or diluent. The composition may be suitable for oral administration or injection.

Transfer factor, specific to a virus, according to the present invention can be used in the treatment of a human or animal body by therapy. More particularly, it can be used to combat or prevent a viral infection attributable to the virus to which it is specific, such as the viruses listed above. Thus transfer factor specific to HSV 1 and HSV 2 has proved particularly successful in treating HSV 1 or HSV 2 infections in mammals, including humans. Diseases that can be treated include herpes genitalis, herpetic keratitis and herpes labialis. A viral infection in a mammal, human or animal, can therefore be combated or prevented by administering to the mammal an effective amount of transfer factor, according to the invention, specific to the virus to which the viral infection is attributable.

The transfer factor is typically administered orally or by injection, for example subcutaneously, intravenously or intramuscularly. The amount of transfer factor administered will depend upon a variety of factors, including the patient under treatment and the severity of the infection. Typically, the transfer factor can be administered at a dose of from 0.01 to 100 units, preferably from 1 to 10 units, for example 5 units, from daily to once a week. This is applicable regardless of the route of administration. 1 Unit is, or corresponds to, the amount of transfer factor obtained by dialysis from $10^8$ cells.

It has also been found that treatment with transfer factor of the invention can be enhanced by its administration in combination with an antiviral agent such as 5-iodo-2'-desoxycytidine. This combination is effective in the treatment of herpetic keratitis in combating not only the ulceration but also opacity formation and neo-vessels.

It is believed that transfer factor specific to a given virus and obtained from liver cells of a mammal, for example in the form of a cell fraction which can be obtained by dialysis, is novel, regardless of its method of preparation. This transfer factor therefore also forms part of the present invention. One advantage of transfer factor obtained from liver cells is that it is much cheaper than transfer factor typically obtained from lymphocytes.

The following Examples illustrate the invention. In the Examples, the Leucocyte Migration Inhibition (LMI) test was carried out as follows:

- 7 -

20 ml of blood are collected in a heparinized tube, and the leucocytes separated by the addition of 20% Plasmagel (Roger Bellon, Paris). The cell pellet is recovered after centrifugation of the supernatant at 300 G for 15 min, and is then washed in saline containing 0.2% heparin (100 i.u./ml.). After a further centrifugation (300 G for 15 min) the pellet is resuspended in 199 medium (Gibco, Glasgow). After an additional centrifugation of 15 min at 100 G, to remove the platelets which remain in the supernatant, the cells are resuspended at $6 \times 10^7$/ml in 199 medium containing 5% by volume foetal calf serum (FCS). This cell suspension is then distributed into heparinized capillary tubes (1.1 mm internal diameter; Proscience, Paris). After centrifugation for 5 min at 300 G the capillaries are cut at the cell interface and each tube is placed in the chamber of a Sterilin migration plate (Polylabo-Paul Block, Strasbourg).

The chambers contain either medium alone, or medium containing herpes antigen HSV 1 or HSV 2 at a concentration of 1/100 (v/v) (Microbiological Associates, Walkersville, Maryland 21793, USA). Preliminary studies showed that this dilution gave the best results. At least three capillaries were used for each antigenic solution. After an incubation of 18 hr at 37°C on a horizontal support, the migration surfaces were projected onto drawing-paper using a photographic enlarger and three diameters of each surface measured. The Migration Index (M.I.) was then calculated using the formula:

$$\text{M.I.} = \frac{\text{Average of the diameters in presence of antigen}}{\text{Average of the diameters in absence of antigen.}}$$

The reaction is statistically positive when the M.I. is lower than 0.8.

Example 1 : Preparation of Transfer Factor specific to
HSV 1 or HSV 2 using guinea pigs

Four guinea pigs were immunized with HSV 1 antigen and four with HSV 2 antigen (Microbiological Associates, Walkersville, Maryland 21793, USA). Three subcutaneous injections of 0.5 ml of viral antigen diluted to 1/100 (v/v) of saline plus 0.5 ml of Complete Freunds Adjuvant (CFA) were given at seven day intervals.

The cellular immunity of the guinea pigs was evaluated by skin testing eight days after the final injection of antigen. The animals were sacrificed once the in vitro tests had shown a satisfactory cellular immunity to the viral antigens. The spleen was removed. Dialysates comprising transfer factor specific to HSV 1 or HSV 2 were prepared following the technique of H.S. Lawrence, "Transfer Factor in Cellular Immunity", The Harvey Lectures, Series 68, p.239 to 350 (1974).

One unit (U) of transfer factor is obtained from $10^8$ cells.

Example 2 : Preparation of Transfer Factor specific to
HSV 1 or HSV 2 using calves

Two calves were immunised once with 1 ml of HSV 1 antigen and 1 ml of HSV 2 antigen (Microbiological Associates, Walkersville, Maryland 21793, USA) respectively.  The antigens were each mixed with 1 ml of CFA for administration.  The cellular immunity of the calves was evaluated 20 days after immunisation.

A LMI was carried out on the 19th day and also a lymphocyte transformation test in the presence of the corresponding herpetic antigen.

The calves were sacrificed on the 21st day.  The spleen,

lymph. nodes and peripheral blood lymphocytes were taken from the calves.  Dialysates specific for the corresponding antigen were obtained by the Lawrence method.

The dialysates were tested _in vivo_ by injection into mice.  Fifteen mice were divided into three groups of five mice.  The mice of Groups 1 and 2 were each injected with dialysate specific to HSV 1 or HSV 2, respectively,

The mice of Group 3 were injected with saline.  48  Hours after injection skin tests were conducted using 0.1 ml of HSV antigen at a dilution of 1/100 (v/v).  The results are shown in Table 1.

Table 1

| Group | Antigen used for skin test | Reaction |
|-------|---------------------------|----------|
| 1 | HSV 1 | + |
| 2 | HSV 2 | + |
| 3 | HSV 1 | - |
| 3 | HSV 2 | - |

Example 3 : Treatment of Patients

Eight human patients, aged from 26 to 50 years, were treated for up to nine months with bovine dialysates comprising transfer factor specific for HSV 1 or HSV 2. These patients had Herpes type 1 or type 2, recurrent and resistant to previous treatments (e.g. Virustat, Cuterpes, Iduviran, Isoprinosine, Solaskyl, Immunonergon, Laroscorbine i.v., antiherpetic and antipoliomyelitis vaccines).

The dialysate was first given by the intramuscular route, by one weekly injection of 5U. This weekly dose was afterwards divided into daily oral doses. The exception to this protocol was patient E.G. who received injections three times a week (3 x 5 U) after absence of response to the doses initially administered.

The efficacy of the treatment was evaluated by the clinical results : reduction in the number and/or duration of the relapses, their intensity and the number of vesicles. Moreover, monthly blood samples allowed the evaluation of the evolution of the cellular immunity by the LMI test and eventually the increase in the number of white blood cells.

A decrease in the number of relapses was noted for all the patients. Those which occurred were shorter and of lower intensity than the relapses before treatment.

The details of Example 3 are summarised in Table 2 below.

Table 2

| Patient | Sex* | Localisation | Age** | Length of illness** | Attacks | | LMI |
|---|---|---|---|---|---|---|---|
| | | | | | Number of relapses per year | Length of each relapse*** | |
| K.H. | F | Genital HSV2 | 26 | 3/4 | 12 | 10 – 27 | 0.99 |
| G.R. | M | Genital HSV2 | 50 | 4 | 12 – 24 | 10 | 0.98 |
| E.K. | F | Genital/ Fingers HSV2 | 26 | 4 | Continuous for six months | 15 | 0.95 |
| D.D. | F | Genital HSV2 | 28 | 3 | 12 | 4 – 15 | 1.04 |
| Y.D. | M | Genital HSV2 | 29 | 4 | 12 | 6 – 10 | 1.06 |
| E.G. | M | Genital HSV2 | 28 | 4 | 24 – 36 | More than 10 | 1.05 |
| G.D. | F | Labial HSV1 | 25 | 15 | At least 12 | 1 – 10 | 1.10 |
| B.L. | F | Labial HSV1 | 44 | 2 | Strong: 5 Weak: 6 to 8 | 15 – 20 2 – 3 | 1.00 |

Table 2 (continued)

| Patient | Treatment **** | | Response to Treatment | | |
|---------|----------------|------|-----------------------------------------|-----------------------------|--------------|
| | Length | Dose | Number of relapses during treatment | Length of each relapse *** | LMI |
| | | | 1 | ½ | 0.81 |
| K.H. | 4 | Protocol | | | |
| | | | 4 +1 ***** | 3 of ∠ 6 1 of 6 | 0.84 |
| G.R. | 7 | Protocol | | | |
| | | | 1 | 3 | 0.80 |
| E.K. | 6 | Protocol | | | |
| | | | 4 | 3 of < 6 1 of > 10 | 0.84 |
| D.D. | 7 | Protocol | | | |
| | | | 3 +2 ***** | 2 of < 6 1 of 6 | 0.81 |
| Y.D. | 7 | Protocol | | | |
| | | | 11 +1 ***** | 2 of < 6 3 of 6-10 6 of > 10 | 0.76 |
| E.G. | 9 | 1 injection 3 times per week | | | |
| | | | 1 +1 ***** | < 2 | not measured |
| G.D. | 3 | Protocol | | | |
| | | | 1 | < 6 | 0.87[1] |
| B.L. | 6 | Protocol | | | |

- 12 -

0101200

0101200

- 13 -

Notes to Table 2

* : M = male, F = female

** : in years

*** : in days

**** : in months

***** : relapses induced within 24 hours of administration of transfer factor at the beginning of the treatment

The LMI stated under "Response to Treatment" represents the best value obtained for each patient.

An LMI was carried out each month. In general, a decrease in the M.I. was observed, but fluctuations for the same patient from one month to the next were also frequent.

For E.K., the taking of the first dose of transfer factor was followed by the disappearance of the crises which had been continuous for several months, very painful and intense, hindering the walking of the patient. One relapse of feeble intensity and very short duration occurred after four months of treatment. Parallel to this clinical evolution, the LMI showed a slow but clear increase in cellular immunity which was translated by a decrease in the M.I..

For B.L., the relapses of recurrent labial herpes during three years, in the form of the classical· "cold-sore", were accompanied in 1981 by a perilesional phlegmonous erysipeloid dermitis. Only one relapse, purely labial, occurred after the commencement of treatment:

the onset suggested an evolution to a dermitis, but the latter did not occur. The LMI study showed a constant and progressive decrease of the M.I..

For other patients, the amelioration was more progressive and the evolution punctuated by a few relapses, although of lower intensity and shorter duration than before treatment.

For G.R., his M.I. decreased slowly but regularly under the effect of treatment until the moment of the first heavy relapse of six days, during which a transitory increase in the M.I. could be observed. The relapses of this patient decreased in duration and in number and became painless under the effect of treatment. However, a recrudescence of the number of relapses with painful symptoms occurred as soon as the treatment was discontinued (3 attacks in 15 days).

Immediately after the treatment had been completed, a placebo was administered to K.H., G.R., E.K., D.D., Y.D., and B.L. for a further three months each. The placebo was administered at daily intervals and consisted either of capsules of glycine or of a freeze-dried glycine powder. The placebo was taken orally. The duration of lapses, calculated as days per month, whilst the placebo was being administered was as follows:

| K.H. | G.R. | E.K. | D.D. | Y.D. | B.L. |
|------|------|------|------|------|------|
| 0    | 16.6 | 2.6  | 7.6  | 0    | 0    |

Example 4: Treatment of keratitis

Three groups of ten rabbits were treated either with transfer factor dialysates specific to HSV 1 (Group A); with an antiviral agent : 5-iodo-2'desoxy-cytidine (IDC) (Group B); or with a combination of transfer factor dialysates specific to HSV 1 and IDC (Group C); while a fourth group, the controls, only received applications of vaseline oil (Group D). Transfer factor was injected sub-conjunctivally (0.5 ml containing 3 U) three times per week during two weeks. The treatment was begun

- 15 -

three days after innoculation of herpes virus type 1 into the right eye. The antiviral was administered in the form of a gel and the controls received the vaseline oil in drops. All the rabbits received drops of neomycin to prevent secondary infections.

The evolution of the keratitis in the different groups was evaluated by the presence of secretions and hyperhemy on the one hand, and by examination using a slit-lamp which allowed the measurement of the severity of ulceration, the number of the opacities and neo-vessels on the other. The animals were examined three times a week.

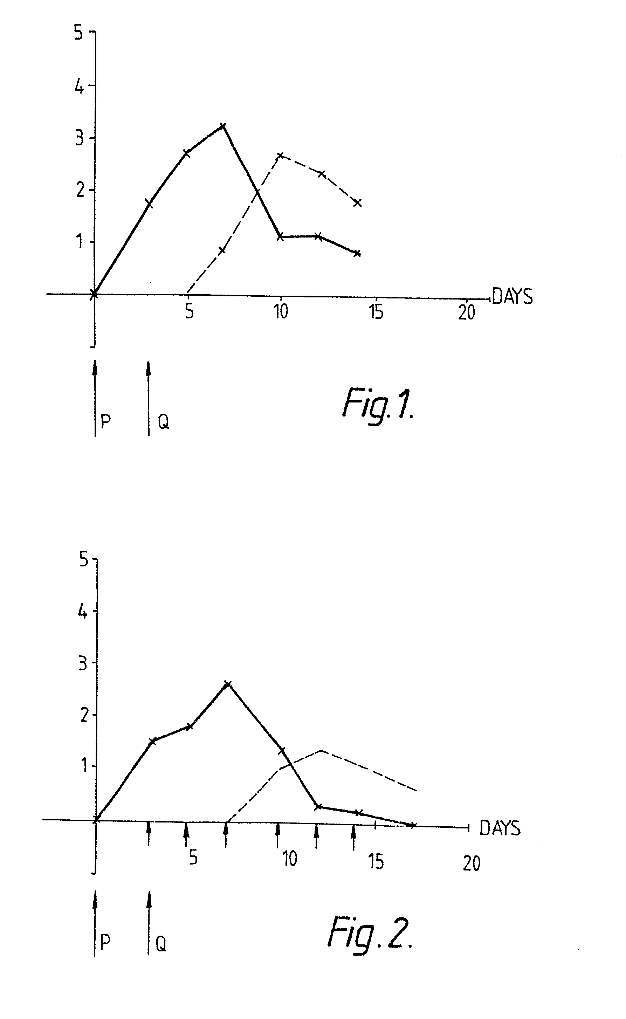

The results have been represented graphically in Figures of the accompanying drawings. Figures 1, 2, 3 and 4 show the results for groups D, A, B and C, respectively. In all Figures:

the x-axis represents time in days;

the y-axis represents the area of lesions;

P represents the administration of the HSV 1;

Q represents the commencement of treatment;

the unbroken line represents ulceration; and

the broken lines represents opacities.

In control group D (Figure 1), the evolution of the herpetic keratitis occurred in two phases:

(a) the first comprised the appearance and growth of ulcers, which attained a maximum on the sixth day; and

- 16 -

(b) during the second, the ulcers regressed and opacities and the neo-vessels appeared. On the fifteenth day, these three lesions persisted.

In group A (Figure 2), the first phase occurred in practically the same way as in the control group. However, from the twelfth day the surface area of the opacities was decreased in relation to the control group. On the fourteenth day, a significant decrease in the number of neo-vessels could be noted ($p < 0.01$).

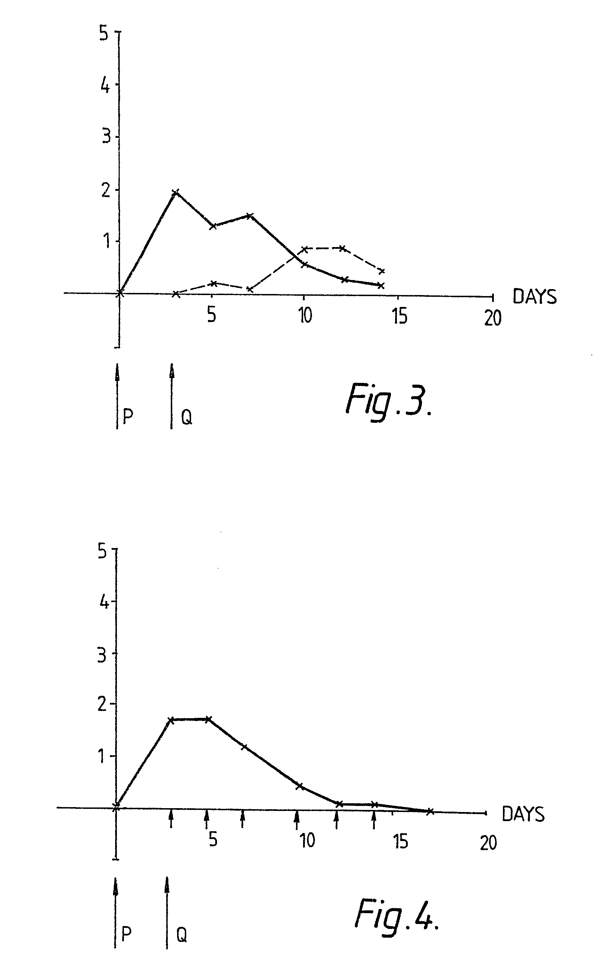

In group B (Figure 3), the area of the ulcerated surface and its evolution were decreased in comparison to the control group. Furthermore a significant decrease in the surface of the opaque area from the twelfth day ($p < 0.005$) as well as in the number of neo-vessels ($p < 0.02$) could be noted.

In group C (Figure 4) no difference in the extent of the ulceration was noted in comparison to group B. However, there was a significant difference ($p < 0.05$) in the opaque surface area with the group B. In effect, from the tenth to twelfth days, there were practically no opaque surfaces in the animals of group D and none presented any neo-vessels.

The results suggest that alone the transfer factor specific to HSV 1 may not influence the evolution of the viral disease as such, but that it plays a role at the level of the sequelae (opacity, neo-vessels). The association of the transfer factor and the antiviral IDC gives the best results. Indeed, not only can a decrease in the ulceration be observed, but also a decrease in the opacities and a total disappearance of the neo-vessels.

In the Figures the values 1 to 5 on the y-axis represent the area of lesions as follows:

1 : surface of lesions is less than one quarter of the corneal surface;

2 : surface of lesions is one quarter of the corneal surface;

3 : surface of lesions is one half of the corneal surface;

4 : surface of lesions is three quarters of the corneal surface; and

5 : the total corneal surface is covered with lesions.

Example 5 : Treatment of Patients

Four human patients suffering from labial HSV 1 or genital HSV 2 were treated with bovine dialysates obtained from liver cells. The dialysates were in the form of capsules or freeze-dried powder and were administered 3 times a week, orally so that 5 U transfer factor were administered per week. The dialysates were specific to HSV 1 or HSV 2 depending upon the afflication of the patient. After the treatment phase a placebo phase was initiated for patients 3 and 4 in which glycine was administered in the same way and at the same frequency as the transfer factor in the treatment phase. The results are shown in Table 3.

- 18 -

## Table 3

Patient

| No. | Sex | Illness | Length of Illness (years) | Duration of Relapses (days/month) |
|---|---|---|---|---|
| 1 | F | genital HSV 2 | 5 | 5.83 |
| 2 | F | genital HSV 2 | 5 | 15.0 |
| 3 | F | genital HSV 2 | 2 | 12.5 |
| 4 | F | labial HSV 1 | 2 | 7.3 |

| Patient No. | Treatment Phase | | Placebo Phase | |
|---|---|---|---|---|
| | Duration (months) | Duration of Relapses (days/month) | Duration (months) | Duration of Relapses (days/month) |
| 1 | 6 | 0.5 | – | – |
| 2 | 5 | 5.83 | – | – |
| 3 | 2 | 6.0 | 4 | 6.0 |
| 4 | 5 | 0.8 | 3 | 0.8 |

- 19 -

Example 6 : Preparation of Transfer Factor specific
            to Cytomegalovirus (CMV) using guinea pigs.

Four guinea pigs were immunised with inactivated CMV. Three subcutaneous injections of 0.5 ml of the CMV diluted to 1/100 (v/v) of saline plus 0.5 ml of CFA were given at seven day intervals. The cellular immunity of the animals was evaluated by skin testing. The guinea pigs were sacrificed once the skin tests demonstrated that a satisfactory immunity to viral antigens had been achieved. The liver was removed from each animal. Dialysates comprising transfer factor specific to CMV were prepared by the Laurence method as described in Example 1.

The dialysates were tested in vivo, by injection into mice. Ten mice were divided into two groups of five mice. The mice of Group 1 were injected with the dialysate comprising transfer factor specific to CMV obtained above. The mice of Group 2 were injected with saline. 48 Hours after injection skin tests were conducted using 0.1 ml of the inactivated CMV preparation at a dilution of 1/100 (v/v). The results are shown in Table 4.

Table 4

| Group | Reaction |
|-------|----------|
| 1     | +        |
| 2     | −        |

Blood was collected from the mice of Group 1 and the LMI test as described above was effected. The reaction was positive, showing that the dialysates of transfer factor specific to CMV had transferred cell-mediated immunity to the naive leucocytes of the mice of Group 1. The percentage inhibition in the LMI test was 75-80%.

Example 7 : Preparation of Transfer Factor specific to
measles virus (MV) using monkeys.

Four guinea pigs were immunised with inactivated
MV. Liver dialysates comprising transfer factor
specific to MV were prepared by the procedure of
Example 6. The dialysates were tested in vivo by
injection into mice in the same manner as Example 6,
except that the mice of Group 1 were injected with the
dialysates comprising transfer factor specific to MV
and the skin tests were conducted using 0.1 ml of the
inactivated MV preparation. The results of the skin
tests are shown in Table 5.

Table 5

| Group | Reaction |
|-------|----------|
| 1     | +        |
| 2     | −        |

Blood was collected from the mice of Group 1
and the LMI test as described above was effected. The
reaction was positive, showing that the dialysates of
transfer factor specific to MV had transferred cell-
mediated immunity to the naive leucocytes of the mice
of Group 1. The percentage inhibition in the LMI
was 76%.

Example 8 : Preparation of Transfer Factor specific to
hepatitis B virus (HBV) using guinea pigs

Four guinea pigs were immunised with HBV. Liver
dialysates comprising transfer factor specific to HBV
were prepared by the procedure of Example 6. The
dialysates were tested in vivo by injection into mice
in the same manner as Example 6, except that the mice
of Group 1 were injected with the dialysates comprising
transfer factor specific to HBV and the skin tests were
conducted using 0.1 ml of the HBV preparation.
The results of the skin tests are shown in Table 6.

- 21 -

### Table 6

| Group | Reaction |
|-------|----------|
| 1 | + |
| 2 | - |

Blood was collected from the mice of Group 1 and the LMI test as described above was effected. The reaction was positive, showing that the dialysates of transfer factor specific to HBV had transferred cell-mediated immunity to the naive leucocytes of the mice of Group 1. The percentage inhibition in the LMI test was 75%.

In Example 6 the inactivated CMV was a commercially available preparation obtained from Dynatech Laboratories Ltd, Billingshurst, Sussex, GB. In Example 7 the inactivated MV was a commercially available preparation obtained from Hoechst-Behring, France. In Example 8 the HBV consisted of viral particles extracted from the serum of hepatitis B positive patients.

**Example 9 : Preparation of Transfer Factor specific to Chickenpox using guinea pigs**

Two guinea pigs were each immunised with 0.5 ml of a chickenpox virus preparation (Hoechst-Behring, code RYP) and liver cell dialysates prepared according to the procedure of Example 6. The dialysates were tested *in vivo* by injection into mice in the same manner as in Example 6, except that the mice of Group 1 were injected with the dialysates prepared above comprising transfer factor specific to chickenpox and the skin test were conducted using 0.1 ml of the chickenpox virus preparation. The results are shown in Table 7:

### Table 7

| Group | Reaction |
|-------|----------|
| 1 | + |
| 2 | - |

0101200

- 22 -

C L A I M S

1. A process for the preparation of transfer factor specific to a given virus, which process comprises immunising a mammal with respect to the virus and obtaining the transfer factor thus-produced specific to the virus from the immunised mammal.

2. A process according to claim 1 in which the virus is a herpesvirus, myxovirus, hepatitis virus or pathogenic animal virus.

3. A process according to claim 2 in which the virus is herpes simplex virus 1, herpes simplex virus 2, a measles virus, cytomegalovirus, herpes zoster virus, hepatitis B virus, an influenza virus, rubella virus or african swine fever virus.

4. A process according to any one of the preceding claims in which the transfer factor is obtained in the form of a cell fraction, derived from cells of lymphoid origin or from liver cells, containing substantially no molecules with a molecular weight of more than 12000.

5. A process according to claim 4 in which the cell fraction is a cell dialysate.

6. A process according to any one of the preceding claims in which the transfer factor once obtained is lyophilised.

7. Transfer factor, specific to a given virus and prepared by a process as claimed in any one of the preceding claims, for use in a method of treatment of a human or animal body by therapy.

8. Transfer factor specific to a given virus and obtained from liver cells of a mammal.

9. A pharmaceutical composition comprising, as active ingredient, transfer factor prepared by a process as claimed in any one of claims 1 to 6 or which is as claimed in claim 7 or 8, together with a pharmaceutically acceptable carrier or diluent.

Fig.1.

Fig.2.

Fig.3.

Fig.4.